**Europäisches Patentamt**

**European Patent Office**

**Office européen des brevets**

(11) Veröffentlichungsnummer: **0 237 981**
**A2**

(12) **EUROPÄISCHE PATENTANMELDUNG**

(21) Anmeldenummer: 87103678.6

(22) Anmeldetag: 13.03.87

(51) Int. Cl.⁴: **A61K 35/16** , A61K 37/02 , C07K 3/12 , A61L 2/10

(30) Priorität: 20.03.86 DE 3609431

(43) Veröffentlichungstag der Anmeldung:
23.09.87 Patentblatt 87/39

(84) Benannte Vertragsstaaten:
AT BE CH DE ES FR GB GR IT LI LU NL SE

(71) Anmelder: **Biotest Pharma GmbH**
**Flughafenstrasse 4**
**D-6000 Frankfurt 71(DE)**

(72) Erfinder: **Kotischke, Ronald, Dr., Dipl.-Chem.**
**Kleiststrasse 23**
**D-6072 Dreieich(DE)**
Erfinder: **Stephan, Wolfgang, Dr., Dipl.-Chem.**
**Phil.-Holzmann-Strasse 84**
**D-6072 Dreieich(DE)**
Erfinder: **Piechaczek, Detlef, Dr., Dipl.-Chem.**
**Darmstödter Strasse 54**
**D-6115 Mönster(DE)**
Erfinder: **Dichtelmüller, Herbert, Dr.,**
**Dipl.-Biologe**
**Rossertstrasse 14**
**D-6231 Sulzbach(DE)**
Erfinder: **Klein, Herwald, Dr., Dipl.-Chem.**
**Buchenweg 1**
**D-6108 Weiterstadt(DE)**

(74) Vertreter: **Beil, Hans Christoph, Dr. et al**
**Beil, Wolff und Beil Rechtsanwälte**
**Adelonstrasse 58**
**D-6230 Frankfurt am Main 80(DE)**

(54) Verfahren zur Herstellung eines den Blutgerinnungsfaktor VIII enthaltenden, sterilen Präparates.

(57) Die Erfindung betrifft ein Verfahren zur Herstellung eines den Blutgerinnungsfaktor VIII enthaltenden, sterilen Präparates mit einer spezifischen Aktivität von mindestens 5 I.E. Faktor VIII pro mg Protein und einer verbesserten Thrombinaktivierbarkeit aus menschlichem Blutplasma durch Fraktionierung von Kryopräzipitat, Sterilisation, Ankonzentrierung, Sterilfiltration und gegebenenfalls Gefriertrocknung, bei dem man Kryopräzipitat in einem NaCl, Glycin und epsilon-Aminocapronsäure enthaltenden Puffermedium mit hoher Ionenstärke löst, das gelöste Kryopräzipitat mit 0,01 bis 0,06 g $\beta$-Propiolacton pro g Protein behandelt, aus der Lösung durch Zugabe von Ethanol bei einer Ethanolkonzentration von 10 %, bezogen auf die Lösung, und einem pH-Wert von 5,9 Fibrinogen, Fibronectin und andere Begleitproteine ausfällt, nach Abtrennen des Niederschlages aus der verbleibenden Lösung den Faktor VIII mit Ammonsulfat bei einer Molarität von 1,3 M ausfällt und den ausgefällten, abgetrennten Faktor VIII in einem NaCl, Glycin und Lysin enthaltenden Puffermedium löst und mit UV-Licht einer Intensität von 1 mWatt/cm² ˣ Min bestrahlt.

## Verfahren zur Herstellung eines den Blutgerinnungsfaktor VIII enthaltenden, sterilen Präparates.

Die Erfindung betrifft das in den Patentansprüchen beschriebene Verfahren zur Herstellung eines den Blutgerinnungsfaktor VIII enthaltenden, sterilen Präparates mit einer spezifischen Aktivität von mindestens 5 I.E. Faktor VIII pro mg Protein und einer verbesserten Thrombinaktivierbarkeit.

Das erfindungsgemäß hergestellte Faktor VIII-Präparat eignet sich als Mittel zur Behandlung der Hämophilie A.

Es sind bereits zahlreiche Verfahren zur Herstellung von Präparaten, welche den Blutgerinnungsfaktor VIII (= Antihämophilie A bzw. AHF, hier auch als Faktor VIII bzw. F. VIII bezeichnet) enthalten und zur Therapie der Hämophilie A eingesetzt werden, bekannt. Zu den bekannten Faktor VIII-Präparaten gehören auch Konzentrate mit unterschiedlichen spezifischen Aktivitäten.

Besondere Bedeutung bei der Faktor VIII-Konzentrat-Herstellung kommt den beiden folgenden Fragen zu:

1) Wie läßt sich der Faktor VIII mit ausreichender Ausbeute so isolieren, daß das potentielle Risiko der Virusübertragung ausgeschlossen wird?

2) Ist durch die zur Faktor VIII-Isolierung, Reinigung und Sterilisation angewandten Verfahren gewährleistet, daß die biologische F. VIII-Funktion und die in vivo Recovery ausreichend erhalten bleiben?

Jeder Verfahrensschritt bei der Isolierung und Reinigung wertvoller Proteine aus humanem Plasma, der nicht mit einer Ausbeute von 100 % arbeitet, führt sehr schnell zu einer gravierenden Verschlechterung der gesamten Ausbeute, selbst wenn bei jedem Verfahrensschritt die Ausbeute noch 90 % beträgt.

Für die Faktor VIII-Herstellung aus humanem Plasma dient heute üblicherweise Kryopräzipitat als Ausgangsmaterial für die F. VIII-Reinigung, wobei ein 1965 von Pool & Shannon (Pool, J.G., Shannon, A.E.: Production of high-potency concentrates of antihemophilic globulin in a closed-bag system, New England Journal of Medicine 273 (1965), 1443) beschriebenes Verfahren zur Anwendung kommt, bei dem im technischen Maßstab nur etwa 50 % der im Plasma vorhandenen Faktor VIII-Aktivität erhalten werden.

Durch den Zusatz von Heparin zu Plasma läßt sich zwar die Faktor VIII-Ausbeute bei diesem Verfahrensschritt erhöhen (vgl. EP-PSen 0 033 582, 0 053 046 und 0 077 119 sowie Smit Sibinga, C.T., Welbergen, H. und Das, P.C.: High yield method of production of freeze-dried purified factor VIII by blood banks, The Lancet Nr. 8244 (1981), 449-450). Der Heparin-Zusatz zu Plasma bringt jedoch ein gravierendes Problem mit sich, so daß sich dieses Verfahren bis heute nicht in großem Maßstab hat durchführen lassen. Nachdem das Kryopräzipitat aus dem Plasma entfernt wurde, muß nämlich aus ökonomischen Gründen das verbleibende Plasma nach den üblichen Fraktionierverfahren zu weiteren Produkten verarbeitet werden. Erfahrungen über die Aufarbeitung von mit Heparin versetztem Plasma liegen für die Plasmafraktionierung jedoch nicht vor, da diese Verfahren alle mit Citrat stabilisierte Plasmen verarbeiten.

Kryopräzipitat besteht im wesentlichen aus Fibrinogen, Fibronectin, Albumin, IgG und IgM, während der Faktor VIII weniger als 1 % des gesamten Proteins ausmacht. Die spezifische Aktivität des Faktor VIII C von Kryopräzipitaten beträgt normalerweise etwa 0,1 bis 0,3 Einheiten Faktor VIII/mg Protein. Nach einer bereits 1946 von Cohn u.a. eingeführten Methode wird der Faktor VIII aus Plasma, zusammen mit Fibrinogen, bei einer Ethanolkonzentration von 8 % (V/V) bei pH 7,0 -7,2, in der Kälte ausgefällt (Cohn, E.J., L.E. Strong, W.L. Hughes, D.J. Mulford, J.N.Ashworth, M. Melin und H. Taylor: A system for the separation into fractions of the protein and lipoprotein components of biological tissues and fluids, Journal of the American Chemical Society 68 (1946), 459). Blombäck hat diese Cohn I-Fraktion durch Extraktion mit einer Ethanol-Glycin-Citrat-Mischung weiter gereinigt, wobei der Faktor VIII und das Fibrinogen im Ethanol-Niederschlag verblieben (Blombäck, M: Purification of antihemophilic globulin. I.Some studies on the stability of the antihemophilic globulin activity in fraction I -0 and a method for its partial separation from fibrinogen, Arkiv för Kemi 12 (1958), 387).

Die Möglichkeit, den Faktor VIII mit etwa 2 M Glycin, bei niedrigen Temperaturen zu präzipitieren, ist bekannt durch Webster u.a., Amer. J. Med. Sc. 250, Nr. 6 (1965), 643-650. Webster verwendete in Wasser gelöstes Kryopräzipitat und fraktionierte bei einer Temperatur unter 10°C, mit zunehmender Glycinkonzentration bis 2,3 M. Faktor VIII-Präparate, die durch Glycin-Fällungen erhalten werden, haben eine spezifische Aktivität von 0,3 bis 0,5 Einheiten Faktor VIII/mg Protein.

In der EP-PS 0 032 655 ist ein Verfahren beschrieben, bei dem Glycin nicht als Fällungsmittel für den Faktor VIII verwendet wird, sondern der Faktor VIII bei einer Glycinkonzentration von 1,5 M bei einer Temperatur von mindestens +15°C und einem pH-Wert von 6,3 bis 7,8 in Lösung bleibt. Der Faktor VIII wird aus der glycinhaltigen Lösung mit Salzen, vorzugsweise 2 M NaCl, ausgefällt. In der WO 84/03628 ist ein Verfahren beschrieben, bei dem während der Faktor VIII-Präzipitation mit Polyethylenglykol (PEG) Aminosäuren als "salting-in" Mittel dienen.

Verfahren zur Reinigung des Faktor VIII aus Kryopräzipitat oder der Cohn I-Fraktion, bei denen mehrere Präzipitationsschritte zur Anwendung kommen, sind ebenfalls bekannt (vgl. DE-AS 17 67 285, DE-PS 31 29 987 und US-PS 3 631 018 sowie E. Bidwell, G.W.R. Dike und W.H. Ford, Therapeutic Materials in Human Blood Coagulation, Haemostasis und Thrombosis, Verlag Blackwell Scientific Pubications (1972), 225-249, Oxford, London, Edingburgh, Melbourne).

Seit Mitte der 70iger Jahre die Hepatitis B Marker diagnostizierbar wurden, stellte sich die Übertragung der Hepatitis B durch Konzentrate der Gerinnungsfaktoren bei der Hämophilie-Behandlung als eine wesentliche unerwünschte Nebenwirkung heraus (Grady, G.F.: Transfusion and Hepatitis: Update in 78 New Engl. J. Med. 298 (1978), 1413; und Trepo, C., O. Hantz, M.F. Jacquier, G. Nemoz, R. Cappel und D.Trepo: Different Fates of Hepatitis B Virus Markers during Plasma Fractionation. A Clue to the Infectivity of Blood Derivatives, Vox Sang. 35 (1978), 143).

Außer der Hepatitis B kann auch noch die Hepatitis Non-A, Non-B durch Blut und Blutprodukte übertragen werden. Bei der Hepatitis Non-A, Non-B kommt erschwerend hinzu, daß bisher keine diagnostische Routine-Methode entwickelt werden konnte, um die entsprechenden serologischen Marker zu erfassen. Seit 1982 sind die ersten Fälle von AIDS unter Hämophilen bekannt geworden. Inzwischen ist das Risiko der AIDS-Übertragung durch Blut und Blutprodukte neben dem Risiko der Hepatitis-Übertragung zu der wichtigsten unerwünschten Nebenwirkung dieser Präparate bei der Hämophilie-Behandlung geworden - (Editorial: Blood Transfusion, Haemophilia, and AIDS, Lancet II (1984), 1433-1435; AIDS Center News, Dez. 1985, Bd. 2, Nr.4, Status of Transfusion Therapy).

Da es durch diagnostische Maßnahmen nicht gelingt, Blutprodukte ohne das Risiko einer Virusübertragung herzustellen, ist die Sterilisation von Blut und Blutbestandteilen ein Weg, dieses Risiko zu umgehen. Aus der DE-PS 30 33 932 ist ein Kaltsterilisationsverfahren bekannt, bei dem vier verschiedene Sterilisationsmaßnahmen, nämlich eine Behandlung mit einem nichtionogenen Tensid, eine Bestrahlung mit UV-Licht, eine Behandlung mit β-Propiolacton (β-PL) und eine Adsorptionsbehandlung mit kolloidaler Kieselsäure, angewendet werden, um ein sicheres F. VIII-Produkt zu erhalten.

Der Erfindung liegt die Aufgabe zugrunde, ein Verfahren zur Herstellung eines den Blutgerinnungsfaktor VIII enthaltenden, sterilen Präparates mit einer spezifischen Aktivität von mindestens 5 I.E. Faktor VIII pro mg Protein und einer verbesserten Thrombinaktivierbarkeit aus menschlichem Blutplasma durch Fraktionierung von Kryopräzipitat, Sterilisation, Ankonzentrierung, Sterilfiltration und gegebenenfalls Gefriertrocknung bereitzustellen, bei dem durch die Fraktionierung unter Anwendung verbesserter Puffermedien und Stabilisatoren das Präparat so gereinigt wird, daß mit Hilfe von nur zwei Sterilisationsmaßnahmen eine ausreichende Virusinaktivierung sichergestellt und die biologische Wirksamkeit des Faktor VIII-Proteins möglichst wenig beeinträchtigt wird.

Diese Aufgabe wird erfindungsgemäß dadurch gelöst, daß man Kryopräzipitat in einem NaCl, Glycin und epsilon-Aminocapronsäure enthaltenden Puffermedium mit hoher Ionenstärke löst, das gelöste Kryopräzipitat mit 0,01 bis 0,06 g β-Propiolacton pro g Protein behandelt, aus der Lösung durch Zugabe von Ethanol bei einer Ethanolkonzentration von 10 %, bezogen auf die Lösung, und einem pH-Wert von 5,9 Fibrinogen, Fibronectin und andere Begleitproteine ausfällt, nach Abtrennen des Niederschlages aus der verbleibenden Lösung den Faktor VIII mit Ammonsulfat bei einer Molarität von 1,3 M ausfällt und den ausgefällten, abgetrennten Faktor VIII in einem NaCl, Glycin und Lysin enthaltenden Puffermedium löst und mit UV-Licht einer Intensität von 1 mWatt/cm² × Min bestrahlt.

Mit Hilfe des erfindungsgemäßen Verfahrens wird ein hochgereinigtes und sterilisiertes Faktor VIII-Konzentrat mit einer spezifischen Aktivität von mindestens 5 I.E. Faktor VIII pro mg Protein erhalten, welches darüberhinaus eine überraschend hohe Thrombinaktivierbarkeit, nämlich eine Steigerung der Faktor VIII-Aktivität durch Thrombin-Aktivierung auf mindestens das 10-fache, sowie eine überraschend verlängerte biologische Halbwertszeit von über 15 Stunden aufweist. Die biologische Halbwertszeit des F. VIII:C nach der Transfusion an Hämophilen beträgt für bisher bekannte F. VIII-Präparate 8 -12 h (T.S. Zimmermann und D. Meyer: Structure and function of factor VIII/von Willebrand factor in Haemostasis and Thrombosis, Verlag Churchill Livingston 1981, 111-123).

Die Erfindung beruht auf mehreren überraschenden Befunden. Im Gegensatz zu den bekannten Fraktionierverfahren, bei denen der Faktor VIII zusammen mit Fibrinogen und Fibronectin aus einem Milieu mit niedriger Ionenstärke ausgefällt wird, ist nach dem erfindungsgemäßen Verfahren der Faktor VIII bei hoher Ionenstärke (1,0 M NaCl, 0,2 M Glycin und 0,1 M epsilon-Aminocapronsäure) nach einer Behandlung mit β-Propiolacton nicht mit Ethanol bei einer Ethanolendkonzentration von 10 % ausfällbar. Überraschenderweise werden nur die im Kryopräzipitat vorhandenen Begleitproteine, wie das Fibrinogen und Fibronectin, bei dieser Ethanolkonzentration ausgefällt. Der Faktor VIII wird anschließend aus der verbleibenden ethanolhaltigen Lösung mit Ammonsulfat (AMS) bei 1,3 M AMS und pH 6,4 ausgefällt. Der in einem Puffersystem aus 1,0 M NaCl, 0,1 M Glycin und 0,02 M Lysin, bei pH 7,5 aufgelöste F. VIII-Niederschlag enthält den Faktor VIII in einer gegenüber Plasma um einen Faktor von größer als 250 gereinigten Form.

Auf die Problematik der Kaltsterilisation des Faktor VIII in Plasma und von Kryopräzipitat mit β-Propiolacton und UV-Bestrahlung wurde in der DE-PS 30 33 932 hingewiesen. Der in dieser Patentschrift gewählte Weg zur Umgehung dieser Probleme beinhaltet vier verschiedene Sterilisationsmaßnahmen. Die bei diesem Verfahren erforderliche UV-Dosis beträgt 2 mWatt/cm² × Min. Die nachstehende Tabelle enthält die Ergebnisse einer Vergleichsuntersuchung der Effektivität der UV-Sterilisation, unter den Bedingungen, die bisher verwendet wurden und den erfindungsgemäßen Bedingungen einer auf die halbe Dosis reduzierten UV-Bestrahlung bei der Anwendung auf die hochgereinigte Faktor VIII-Lösung.

## Tabelle

### Vergleich der Effektivität der UV-Sterilisation

#### Inaktivierung in log 10-Stufen

| | Kyropräzipitat 2 mWatt/cm²xMin (Vergleich) | gereinigte F VIII-Lösung 1 mWatt/cm²xMin (erfindungsgemäß) |
|---|---|---|
| Hepatitis A | 4 | 7 |
| HTLV III | 2,5 | >3,5 |

Bei den beiden miteinander verglichenen Verfahren wurde eine identische β-Propiolactonkonzentration angewendet. Überraschend bei diesem Ergebnis ist der Befund, daß die UV-Dosis bei der Sterilisation einer potentiell mit Viren kontaminierten F. VIII-Lösung auf die Hälfte reduziert werden kann und die Effektivität der Virusinaktivierung unter diesen Bedingungen nicht vermindert wird, sondern sogar deutlich verbessert worden ist. Die in der Tabelle aufgeführten Ergebnisse wurden durch Versuche erhalten, bei denen Plasma bzw. Kryopräzipitat bzw. die erfindungsgemäß gereinigte Faktor VIII-Lösung absichtlich mit Hepatitis A-bzw. HTLV III-Viren kontaminiert wurde, da sich an diesen Viren die Wirksamkeit beider Verfahren mit labordiagnostischen Mitteln überprüfen läßt. Diese Versuchsergebnisse belegen, daß durch die Anwendung des erfindungsgemäßen Verfahrens der Faktor VIII-Reinigung aus Kryopräzipitat eine ausreichende Effektivität von zwei Sterilisationsmaßnahmen bei der Verwendung einer hochgereinigten Faktor VIII-Lösung erreicht wird.

Faktor VIII-Thrombinaktivierung

Die Thrombinaktivierung des Faktors VIII ist unter anderem ein Maß für den Grad seiner Reinheit. Das Ausmaß der Faktor VIII-Aktivierbarkeit ist abhängig von seiner Vorgeschichte. Diese Vorgeschichte ist abhängig von dem Ausmaß, in dem Thrombin und andere Proteasen den Faktor VIII vor seiner weiteren Reinigung bereits beeinträchtigt haben. Das Ausmaß, in dem der Faktor VIII mit Thrombin aktivierbar ist, ist somit auch ein Maß für seine Bioverfügbarkeit.

Zur Bestimmung der Thrombinaktivierbarkeit wurden die zu prüfenden F. VIII-haltigen Lösungen mit 0,9 % NaCl auf eine F.VIII C-Aktivität von etwa 1 I.E./ml eingestellt. 1 I.E. Faktor VIII/ml ist durch einen WHO-Standard definiert und entspricht etwa der Aktivität eines normalen Citratplasmapools von mindestens 30 Spendern. Zu der auf etwa 1 I.E./ml eingestellten Faktor VIII-Lösung wurden 0,1 Einheiten Thrombin gegeben und die Faktor VIII C-Aktivität sofort und nach einer Inkubationszeit von 5, 10, 20 und 30 Minuten bestimmt. Die Faktor VIII C-Aktivität des erfindungsgemäß hergestellten Faktor VIII-Konzentrates ließ sich durch Thrombinzugabe um das 10-fache steigern, während das F. VIII-Präparat, das nach dem gleichen Verfahren ohne Sterilisationsmaßnahmen mit β-PL und UV hergestellt wurde, sich in seiner Aktivität mit Thrombin nur auf das 2,5-fache steigern läßt.

Faktor VIII-Halbwertszeitbestimmung

Fünf Hämophilie A-Patienten wurden mit dem nach dem erfindungsgemäßen Verfahren hergestellten F.VIII-Konzentrat behandelt. Die F.VIII-Dosis betrug 20 bis 25 I.E. pro kg Körpergewicht. Die F. VIII-Aktivität im Patientenplasma wurde vor, 5, 15 und 30 Minuten und 1 h, 4 h, 6 h, 12 h, 24 h und 48 h nach der F. VIII-Applikation bestimmt. Die F. VIII C-Aktivität wurde mit dem Einstufen-Test, Zweistufen-Test und photometrisch, unter Verwendung eines Peptidsubstrates bestimmt. Die F. VIII-Halbwertszeit wurde mit einem modifizierten Gauss-Newton-Algorithmus in der Modifikation nach Hartley mit Hilfe eines Computers berechnet. Diese Methode erlaubt die Approximation der Meßpunkte an einer Summe zweier e-Funktionen nach dem Prinzip der kleinsten Quadratsumme. Der Vorteil dieses Verfahrens liegt darin, daß Verteilungs- und Eliminationsphasen nicht willkürlich, sondern rechnerisch voneinander getrennt werden. Das Ergebnis der Halbwertszeitbestimmtung wird dadurch unabhängiger von den Zeitpunkten, zu denen der Faktor VIII C nach der F. VIII-Infusion bestimmt wurde.

Die Faktor VIII-Halbwertszeit des erfindungsgemäß hergestellten F. VIII-Konzentrates betrug bei der Bestimmung an fünf Patienten im Durchschnitt 15,5 Stunden und lag damit in überraschender Weise deutlich über den Werten, die für bisher übliche Faktor VIII-Präparate mit etwa 8 bis 12 Stunden gefunden wurden.

Die erfindungsgemäße Aufarbeitung von Kryopräzipitat führt zu einem Faktor VIII-Konzentrat, dessen spezifische Aktivität mindestens 5 I.E. Faktor VIII/mg Protein beträgt, dessen Thrombinaktivierbarkeit überraschend überdurchschnittlich ist (Anstieg der Faktor VIII-Aktivität auf mindestens das 10-fache nach Thrombinzugabe) und dessen biologische Halbwertszeit überdurchschnittlich ist (uber 15 Stunden). Das Fließschema (Abb. I) veranschaulicht die wesentlichen Verfahrensschritte des erfindungsgemäßen Verfahrens.

Die Erfindung wird durch die nachstehenden Beispiele näher erläutert.

Beispiel 1

A. Kryopräzipitat: Kryopräzipitat wurde bei +37°C unter Rühren mit dem Puffer 1 in Lösung gebracht. Puffer 1 enthielt: 1,0 M NaCl, 0,2 M Glycin, 0,1 M epsilon-Aminocapronsäure. Pro kg Kryopräzipitat wurden 4 kg des Puffers 1 verwendet. Das gelöste Kyropräzipitat wurde mit $\beta$-Propiolacton bei Zimmertemperatur behandelt. Pro g Protein wurden 0,015 g $\beta$-Propiolacton verwendet.

B. Ethanol-Fällung: Die $\beta$-PL-behandelte Lösung wurde mit Puffer 1 verdünnt, der pH-Wert auf 5,9 eingestellt und Fibrinogen, Fibronectin und andere Begleitproteine durch Zugabe von 96%igem Ethanol bis zu einer Ethanolendkonzentration von 10 Gew.-% bei -5°C ausgefällt. Die Lösung wurde zur Abtrennung der ausgefällten Proteine zentrifugiert.

C. Faktor VIII Ausfällung und Lösung: Der Faktor VIII wurde aus der ethanolhaltigen zentrifugierten Lösung durch Zugabe einer konzentrierten Ammonsulfatlösung bis zu einer AMS-Konzentration von 1,3 M ausgefällt. Der mit AMS ausgefällte Faktor VIII wurde durch Zentrifugation von der restlichen Lösung abgetrennt. Der den Faktor VIII enthaltende Niederschlag wurde mit Puffer 2 gelöst. Puffer 2 enthielt:1,0 M NaCl, 0,1 M Glycin und 0,02 M Lysin.

D. UV-Bestrahlung: die UV-Bestrahlung der Faktor VIII-Lösung erfolgte mit Hilfe eines UV-Durchfluß-Bestrahlungsgerätes bei 254 $\mu$m mit 1 mWatt/cm² $^{\times}$ Min.

E. F. VIII-Konzentrat-Herstellung: Die UV-bestrahlte F. VIII-haltige Lösung wurde durch Ultrafiltration auf eine Faktor VIII-Konzentration von 30 I.E. Faktor VIII/ml eingestellt, steril filtriert, abgefüllt und gefriergetrocknet.

Beispiel 2

Kryopräzipitat: Kryopräzipitat wurde bei +37°C unter Rühren mit dem Puffer 1 in Lösung gebracht. Im Unterschied zu Beispiel 1 wurden bei der $\beta$-Propiolactonbehandlung 0,05 g $\beta$-PL pro g Protein verwendet. Die sich anschließenden Schritte der Aufarbeitung des Kryopräzipitates erfolgten nach der Arbeitsweise von Beispiel 1.

Beispiel 3

Kryopräzipitat wurde wie in Beispiel 2 in Lösung gebracht und mit $\beta$-Propiolacton behandelt. Die $\beta$-Propiolacton behandelte Lösung wurde mit Hilfe eines UV-Durchfluß-Bestrahlungsgerätes bei 254 $\mu$m mit 2 m Watt/cm² $^x$ min. bestrahlt.

Die sich anschließenden Schritte der Aufarbeitung des Kryopräzipitates erfolgten nach der Arbeitsweise von Beispiel 1.

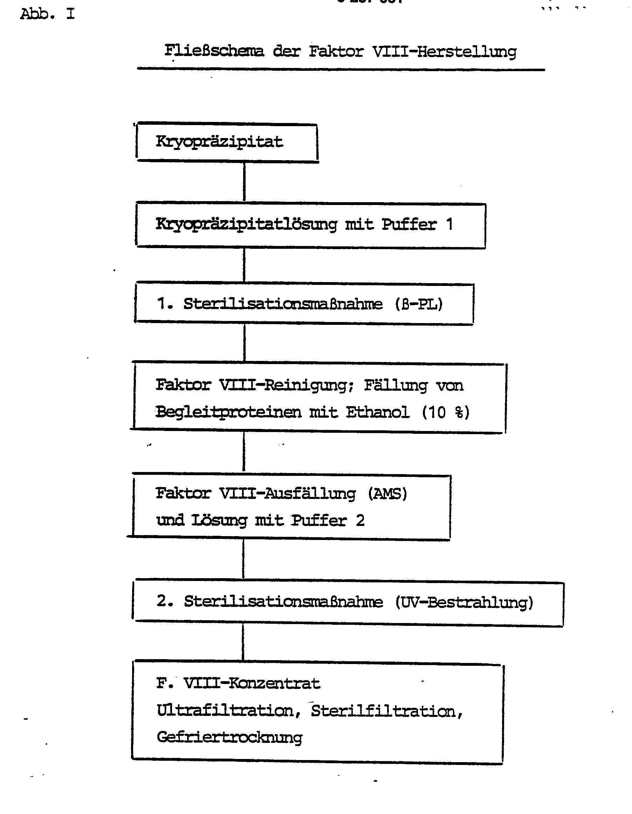

**Ansprüche**

1. Verfahren zur Herstellung eines den Blutgerinnungsfaktor VIII enthaltenden, sterilen Präparates mit einer spezifischen Aktivität von mindestens 5 I.E. Faktor VIII pro mg Protein und einer verbesserten Thrombinaktivierbarkeit aus menschlichem Blutplasma durch Fraktionierung von Kryopräzipitat, Sterilisation, Ankonzentrierung, Sterilfiltration und gegebenenfalls Gefriertrocknung, dadurch gekennzeichnet, daß man Kryopräzipitat in einem NaCl, Glycin und epsilon-Aminocapronsäure enthaltenden Puffermedium mit hoher Ionenstärke löst, das gelöste Kryopräzipitat mit 0,01 bis 0,06 g $\beta$-Propionlacton pro g Protein behandelt, aus der Lösung durch Zusatz von Ethanol bei einer Ethanolkonzentration von 10 %, bezogen auf die Lösung, und einem pH-Wert von 5,9 Fibrinogen, Fibronectin und andere Begleitproteine ausfällt, nach Abtrennen des Niederschlages aus der verbleibenden Lösung den Faktor VIII mit Ammonsulfat bei einer Molarität von 1,3 M ausfällt und den ausgefällten, abgetrennten Faktor VIII in einem NaCl, Glycin und Lysin enthaltenden Puffermedium löst und mit UV-Licht einer Intensität von 1 mWatt/cm² $^x$ Min bestrahlt.

2. Verfahren nach Anspruch 1, dadurch gekennzeichnet, daß man die UV-bestrahlte, Faktor VIII enthaltende Lösung durch Ultrafiltration ankonzentriert und anschließend sterilfiltriert und gefriertrocknet.

3. Verfahren nach Anspruch 1 oder 2, dadurch gekennzeichnet, daß man zum Lösen des Kryopräzipitates ein Puffermedium mit einem Gehalt von 1,0 M NaCl, 0,2 M Glycin und 0,1 M epsilon-Aminocapronsäure verwendet.

4. Verfahren nach einem der Ansprüche I bis 3, dadurch gekennzeichnet, daß man zum Lösen des mit Ammonsulfat ausgefällten Faktors VIII ein Puffermedium mit einem Gehalt von 1 M NaCl, 0,1 M Glycin und 0,02 M Lysin verwendet.

Abb. I

Fließschema der Faktor VIII-Herstellung

```
┌─────────────────────────────┐
│ Kryopräzipitat              │
└─────────────────────────────┘
              │
┌─────────────────────────────┐
│ Kryopräzipitatlösung mit Puffer 1 │
└─────────────────────────────┘
              │
┌─────────────────────────────┐
│ 1. Sterilisationsmaßnahme (ß-PL) │
└─────────────────────────────┘
              │
┌─────────────────────────────┐
│ Faktor VIII-Reinigung; Fällung von │
│ Begleitproteinen mit Ethanol (10 %) │
└─────────────────────────────┘
              │
┌─────────────────────────────┐
│ Faktor VIII-Ausfällung (AMS) │
│ und Lösung mit Puffer 2     │
└─────────────────────────────┘
              │
┌─────────────────────────────┐
│ 2. Sterilisationsmaßnahme (UV-Bestrahlung) │
└─────────────────────────────┘
              │
┌─────────────────────────────┐
│ F. VIII-Konzentrat          │
│ Ultrafiltration, Sterilfiltration, │
│ Gefriertrocknung            │
└─────────────────────────────┘
```